# NOUVEAU FASCICULE DE BREVET EUROPEEN

(11) **EP 0 194 212 B2**
(45) Date de publication et mention de la décision concernant l'opposition: **11.10.1995**
(45) Mention de la délivrance du brevet: 23.05.1990
(21) Numéro de dépôt: 86420040.7
(22) Date de dépôt: 07.02.1986
(51) Int. Cl.: G01N 33/555, G01N 33/559

(54) **Procédé de mise en évidence d'agglutinats érythrocytaires**
Verfahren zum Nachweis von agglutinierten Erythrozyten
Method for detecting agglutinated erythrocytes

(30) Priorité: 08.02.1985 FR 8502010
(43) Date de publication de la demande: 10.09.1986
(73) Titulaire: Fondation pour la recherche de diagnostiques de laboratoire, 3280 Murten (CH)
(72) Inventeur: Lapierre, Yves, F-69600 Oullins (FR)
(74) Mandataire: Liska, Horst, Dr.-Ing.

(56) Documents cités:
- EP-A- 0 039 195
- EP-B- 0 058 780
- DE-A- 1 598 151
- DE-A- 2 035 784
- FR-A- 2 395 780
- GB-A- 2 017 912
- US-A- 3 492 396
- US-A- 3 876 376
- US-A- 4 105 415
- US-A- 4 254 082
- Larsen et al., Methods in Enzymology 70, pp. 315-322, 1980

## Description

La présente invention concerne un procédé pour la mise en évidence d'agglutinats érythrocytaires, fréquemment utilisé en biologie, notamment en immuno-hématologie et transfusion.

On connaît les techniques classiques de mise en évidence de la réaction antigène-anticorps en immuno-hématologie érythrocytaire. Les plus courantes font appel à l'agglutination:
Agglutination spontanée en milieu salin
Agglutination en milieu macromoléculaire
Agglutination d'hématies traitées par des enzymes
Test de Coombs Mourant Race (agglutination après lavage d'hématies revêtues par des anticorps ou du complément, au moyen d'antiglobuline humaine)
Maintien d'une agglutination non spécifique (telle celle induite par l'hexadimethrine bromide ou polybrène®, par exemple)

Méthodes dérivées des procédés cités ci-dessus, ou associant plusieurs d'entre eux (test de Coombs sur hématies trypsinées, test manuel polybrène®-antiglobuline, en deux phases ...).

Ces techniques peuvent généralement être réalisées sur plaque, en tube, ou dans des dispositifs automatiques.

On connaît des développements récents à ces techniques:

Graham décrit en 1982 une méthode originale de séparation sérum-globules, évitant les lavages du test de Coombs classique. Pour cela il utilise, dans un tube particulier, un milieu composé d'albumine et de dextran, de densité intermédiaire entre sérum et globules (Mollison P. L., Blood transfusion in clinical medicine, Blackwell, Oxford, 1983, p. 512). Ortho Diagnostic Systems a commercialisé, sous forme de kit, ce procédé (Simwash®). Il est original, mais, d'après l'auteur, pas plus sensible que le test classique dont il ne diffère pas fondamentalement, dans sa phase finale.

Des techniques dites "en phase solide" voient le jour, avec des méthodologies variables.

La synthèse des deux procédés exposés ci-dessus (séparation sérum-globules sans lavage et phase solide) aurait abouti à la technique suivante: le mélange sérum-globules est centrifugé à travers un liquide de séparation, dans un puits dont le fond est revêtu d'antiglobuline. La lecture est faite automatiquement. Cette méthode, présentée au symposium transfusionnel de Munich (1984) par une équipe parisienne (Muller A. Beige D., Richard D., Matte C., CNTS Paris/Orsay), donnerait d'excellents résultats, en tous cas nettement supérieurs à ceux obtenus par les tests manuels.

La présente invention vise à supprimer certains des inconvénients des méthodes connues, en utilisant un procédé original de mise en évidence de l'agglutination, pour obtenir des tests répondant aux critères suivants:

Praticabilité: Les tests manuels les plus couramment utilisés en immuno-hématologie, s'ils nécessitent des précautions de manipulation et d'interprétation, restent, pour des personnes entraînées, simples et rapides. Le test proposé doit donc répondre aux mêmes exigences de praticabilité. Ceci n'exclut d'ailleurs pas son utilisation dans des systèmes plus ou moins automatisés, pour des applications en séries.

Fiabilité: l'interprétation de la réaction, d'une très grande simplicité doit améliorer la fiabilité de la réponse dans un certain nombre de situations difficiles (réaction faible, urgence, utilisateurs occasionnels...). D'autre part, les précautions à apporter à la manipulation concernant surtout la phase de préparation du milieu, peuvent, dans une certaine mesure, être dissociées, dans le temps, de ces situations difficiles.

Sensibilité: l'amélioration de la sensibilité des tests, du fait des conditions particulières, et du mode de lecture, est primordiale.

Reproducibilité: L'absence, dans la dernière phase, d'intervention humain difficilement quantifiable (selon les tests: lavages manuels, agitation, ajout d'un réactif, temps de lecture...), en réalisant une relative standardisation, doit permettre une meilleure reproductibilité, notamment des titrages.

Dans la phase finale des tests en tubes couramment utilisés en immuno-hématologie, on peut trouver les manipulations suivantes:

Lavages (Coombs et phase "antiglobuline" du test polybrène®-Coombs).

Ajout de réactifs (citrate dans le test manuel au polybrène® antiglobuline dans le test de Coombs ou la phase "antiglobuline" du test polybrène®-Coombs).

Agitation pour remise en suspension des hématies.

Lecture immédiate, par une personne entraînée (ces deux dernières manipulations sont communes à tous les tests d'agglutination en tube, quand la lecture est macroscopique).

Le procédé que nous allons décrire agit sur cette phase. En effet l'incubation ne diffère pas fondamentalement de celle des tests classiques. Cette technique permet toutefois l'utilisation de quantités de réactifs qui sont en moyenne plus faibles que celles utilisées habituellement, notamment pour les hématies-tests, ce qui améliore le rapport sérum/globules pendant l'incubation.

Les lavages, quand ceux-ci sont nécessaires, sont remplacés par une centrifugation douce du mélange sérum-globules à travers un filtre (dans la technique détaillée décrite plus loin, ce filtre est un gel). Les globules, qui sédimentent rapidement, sont alors séparés du sérum. Cet aspect de la technique, s'il est intéressant, n'est pas à proprement parler nouveau, comme signalé précédemment (sauf par le fait que le milieu utilisé est un gel).

L'ajout de réactifs quand il est nécessaire, est remplacé par leur incorporation au milieu de gel: c'est le cas de l'antiglobuline, ou de la solution citratée de resuspension utilisée dans le test manuel au polybrène®. Lors de la centrifugation, on a donc une séparation globules-sérum, mais aussi une réaction avec les molécules incluses dans le filtre.

La taille des pores du filtre constitué par le gel est telle que celui-ci laisse passer les globules libres plus facilement que les agglutinats, ceci lors d'une centrifugation douce, répondant à des critères précis. Une forme particulière, conique, du fond du tube de réaction, en ralentissant la progression des globules lors de la centrifugation, majore le phénomène. La lecture est alors particulièrement aisée: dans les réactions négatives, les globules ont sédimenté complètement, dans les réactions positives, ils restent pris, au moins en partie, dans le filtre de gel. Cette lecture peut sans inconvénient être différée de plusieurs heures, effectuée par plusieurs personnes successivement, photographiée.

Cet aspect du procédé, nouveau, constitue la base de la demande de brevet.

A titre d'exemple non limitatif, on trouvera ci-dessous une description détaillée d'une application: le test à l'antiglobuline.

Préparation du gel. Dans cette application, le filtre est constitué par un gel obtenu de la façon suivante:

Mouiller du Séphadex® G100 Superfine (Pharmacia Fine Chemicals, Upssala, Sweden) avec une solution basse force ionique, à raison de 18 ml de solution par gramme de Séphadex. Dans l'application, la solution basse force ionique utilisée était celle du Centre de Transfusion Sanguine de Lyon, préparée en accord avec la formule de Löw et Messeter (Low B., Messeter L., Antiglobulin test in low-ionic strength salt solution for rapid antibody screening and cross-matching, Vox Sang. 1974, 26:53-61).

Ajouter une antiglobuline contenant 1 g/l de sulfate de dextran (Pharmacia Fine Chemicals, Uppsala, Sweden), à raison d'1 volume pour 10 volumes du mélange Séphadex-solution basse force ionique.

Le gel est préparé simplement, par ajout de la solution basse force ionique au Séphadex ou l'inverse, agitation douce, ajout de l'antiglobuline, nouvelle agitation. Il n'est pas impératif d'utiliser une solution basse force ionique, et des essais concluants ont été réalisés avec d'autres solutions (saline, tampons phosphate, tampons carbonate, solution de resuspension citrate-dextrose de Lalezari (Lalezari P., Jiang A. F., The manual polybrene test: A simple and rapid procedure for detection of red cell antibodies, Transfusion 1980; 20:206-211)).

Il convient par contre d'observer un certain équilibre entre les différentes constituants de l'antiglobuline, pour améliorer la sédimentation des globules libres, et la conservation des réactifs (sérum animal ou albumine, sulfate de dextran). En l'absence de cet équilibre, les globules sont incapables de sédimenter à travers le gel, même si le sérum avec lequel ils ont été incubés ne contient pas d'anticorps. L'antiglobuline utilisée dans l'application était une antiglobuline anti-lgG de chèvre ou de lapin, fabriquée par le Centre de Transfusion Sanguine de Lyon (sérum animal dilué 20 à 50 fois en saline auquel est ajouté du sulfate de dextran pour obtenir une concentration de 1 g/l).

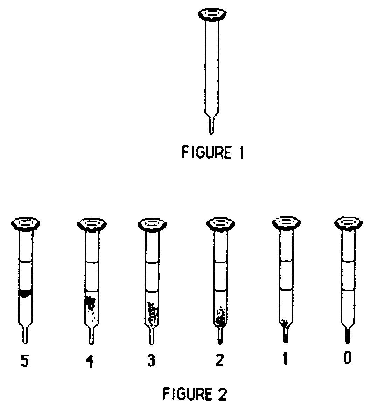

Préparation des tubes à réaction. Les tubes utilisés (tubes pour extraction des micro sédiments réf. 72702 Starstedt, Romnelsdorf, West Germany) sont de petite taille (diamètre intérieur 4 mm, longueur 45 mm) et terminés par une partie conique, prolongée par une pointe (cf. figure 1).

Leur faible capacité permet de travailler avec de petites quantités de réactifs, assure une sédimentation correcte et rend la lecture possible à travers le plastique et le gel. La partie terminale conique, en ralentissant la sédimentation des hématies, d'autant plus qu'elles sont agglutinées, améliore la sensibilité, du test.

D'autres tubes ont été utilisés avec succès, mais celui-ci est particulièrement bien adapté, et donne les meilleures résultats.

Chaque tube reçoit, à la pipette automatique, 200 microlitres de gel, et est ensuite centrifugé brièvement à environ 1000 g (5 secondes, mais cette centrifugation peut sans inconvénient être beaucoup plus longue), pour tasser ce gel.

Réaction. Dans un autre récipient (tube, puits de microplaque...), on mélange 30 microlitres de sérum à 120 microlitres d'une suspension érythrocytaire à 0,25% en solution basse force ionique (Low et Messeter). Après une incubation de 10 minutes à 37°C, on transfère ce mélange à la pipette (Pasteur, par exemple) dans la partie supérieure du tube, au dessus du gel. Peu importe que le contact entre gel et mélange soit immédiat ou non, pourvu que le mode de dépôt n'ait pas fait pénétrer profondément les globules dans le gel.

Le tube est alors centrifugé 10 minutes à 200 unités RCF (radial centrifugation force).
1 unité RCF=(rayon de centrifugation) (vitesse angulaire)²/9,8
9,8 étant la constante de Newton

La vitesse angulaire est exprimée en radian/seconde

Le rayon de centrifugation, exprimé en mètres, est mesuré entre l'axe de la centrifugeuse et le fond du tube.

En cours de centrifugation, la force centrifuge doit être dans l'axe du tube.

La lecture est effectuée directement à la fin de la centrifugation, et reste possible plusieurs heures: les réactions positives sont caractérisées par la rétention d'hématies dans le gel.

Cette rétention est haute et totale pour les réactions fortement positives (figure 2, tubes 3 à 5), plus basse, et parfois incomplète pour les réactions faiblement positives (figure 2, tubes 1 et 2).

Dans les réactions négatives (figure 2, tube 0), tous les globules ont sédimenté dans la pointe.

### Résultats

Titrage d'anticorps immuns irréguliers. Des sérums (donneurs ou malades) correspondant aux principales spécificités anticorpales des systèmes à anticorps irréguliers immuns, ont été titrés par cette technique et par un test de Coombs basse force ionique classique (Low B., Messeter L., Antiglobulin test in low-ionic strength salt solution for rapid antibody screening and cross-matching, Vox Sang. 1974; 26:53-61). Les dilutions des sérums ont été préparées à l'avance, séparées en 2 fractions, numérotées au hasard, et congelées. Les tests ont été réalisés le même jour, après décongélation, par des techniciens différents, travaillant à l'aveugle. Les résultats étaient exprimés en terme de positivité ou négativité, avec essai de cotation de 1 à 5 pour les positifs.

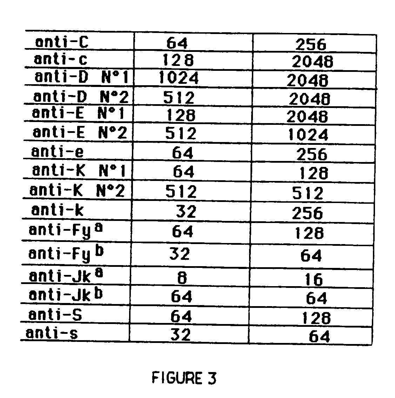

La figure 3 montre les titres obtenus par chacune des méthodes et la supériorité du test sur gel. Il faut noter également qu'aux dernières dilutions positives en test classique correspondaient des réactions extrêmement faibles, visibles seulement par un technicien confirmé effectuant immédiatement la lecture, alors que les dernières positivités, avec le test sur gel, auraient pu être interprétées le lendemain, par une personne n'ayant jamais utilisé cette technique.

Quelques anticorps de nature habituellement IgM (anti-Lewis, anti-M anti-N...) ont été testés, avec des résultats variables.

Recherche de la quantité minimale détectable d'anti-D par titrage d'un sérum étalon. La quantité minimale d'anti-D détectable par cette technique est de 0,4 à 0,8 ng/ml, ce qui correspond à une sensibilité nettement supérieure à celle du test de Coombs, et pratiquement équivalente à celle du test au polybrène® en appareil automatique (Mollison P. L., Blood transfusion in clinical medicine, Blackwell Oxford, 1983, pp. 487-557).

Groupage érythrocytaire, pour les antigènes C, c, D, E, e, K, k, Fy^{a}, Fy^{b}, Jk^{a}, Jk^{b}, S, s. Les sérums utilisés sont des sérums test homologués par le CNRGS, purs ou dilués au 1/2 ou au 1/4. Ces sérums étant suffisamment forts, les réactions sont particulièrement faciles à lire: dans les réactions positives les globules sont tous retenus dans la partie haute du gel. Dans ces conditions, la présence simultanée de globules retenus dans le gel et de globules ayant bien sédimenté, correspond évidemment à une double population. Les hématies minoritaires sont détectables à condition de représenter:

10 à 15% des hématies totales, quand elles ne portent pas l'antigène cherché.

1,5 à 3% des hématies totales, quand elles portent l'antigène cherché. Pour les deux dernières applications présentées (groupage et estimation de la sensibilité), les résultats cités ne correspondent pas aux meilleurs résultats, mais à des résultats habituels.

Dans les conditions d'utilisation décrites ci-dessus, il n'a pas été noté de fausse réaction positive sur les sérums testés. L'utilisation de témoins reste tout de même indispensable.

On peut, à titre d'exemple, citer d'autres applications du procédé décrit:

Test de Coombs direct. Le principe est exactement le même: préparation d'une suspension globulaire à 1% en saline, par exemple, dépôt à la surface du gel, centrifugation, lecture.

Agglutination en milieu salin, ou albumineux. On peut remplacer, dans le gel, le sérum d'animal immunisé par un sérum neutre. Les conditions (temps, température) d'incubation sont alors modifiées en fonction de la nature de l'anticorps mis en jeu.

Test au polybrène®, test mixte polybrène®-antiglobuline. La solution de resuspension remplace alors la solution basse force ionique dans le gel. Le polybrène est, comme dans la technique de Lalezari, ajouté dans le mélange sérum-globules, et la centrifugation induit d'abord une agglutination, puis une désagglutination (sauf réaction positive) quand les globules rencontrent la solution citratée. Dans les cas (anti-Kell) où la désagglutination a lieu malgré la présence d'anticorps, l'antiglobuline contenue dans le gel agit comme il a été décrit précédemment. L'avantage est la relative standardisation des opérations d'agglutination et désagglutination, toujours délicates dans le test manuel au polybrène.

Séparation immunologique des doubles populations. Il est simple, dans ce système, de récupérer, d'une part les globules libres, d'autre par les globules agglutinés.

On peut également citer des modifications possibles de la procédure:

Utilisation d'autres gels (Séphacryl® par exemple), en ne perdant pas de vue que, compte tenu de l'utilisation prévue, le coût doit rester modéré.

Simplification de la procédure. En l'état actuel, le test n'est ni complexe, ni long, mais certaines étapes peuvent être supprimées, notamment le transfert du mélange sérum-globules si ce mélange est réalisé directement dans la partie haute d'un tube réaction dont la partie basse aurait été remplie au préalable par le gel.

Modalités différentes de préparation et conservation du gel, ou de ses constituants.

Intégration dans des systèmes semi-automatiques.

Ce procédé est susceptible d'intéresser tous les laboratoires ayant une activité d'immuno-hématologie, qu'ils soient intra ou extra-hospitaliers.

## Revendications

1. Procédé pour la mise en évidence d'une agglutination érythrocytaire, permettant une lecture facile, directe et prolongée, caracterisé en ce qu'un mélange sérum-hématies est incorporé, après incubation, dans un milieu de gel contenant des anticorps ou non, et que ce mélange est soumis à des conditions particulières de centrifugation, permettant d'un seul trait et en un temps:
a) la séparation du sérum et des hématies à l'intérieur du système de test,
b) le contact des hématies sensibilisées ou non-sensibilisées avec des anticorps contenus dans le milieu de gel,
c) la rétention relative des hématies agglutinées par le gel par rapport aux hématies non agglutinées qui sédimentent.

2. Procédé selon la revendication 1, caractérisé en ce que le milieu de gel est préparé par reconstitution dans un tampon physiologique.

3. Procédé selon la revendication 1, caractérisé en ce que le gel est à base de dextran.

4. Procédé selon la revendication 2, caractérisé en ce que la conservation des anticorps dans le gel et la séparation des hématies agglutinées et non-agglutinées sont améliorées, par l'ajout d'albumine, de dextrans, de polyvinylpyrrolidone ou de sérums animaux.

5. Procédé selon la revendication 2, caractérisé en ce que le milieu de gel comprend une antiglobuline humaine comme anticorps.

## Patentansprüche

1. Verfahren zum Nachweis einer Erythrocyten-Agglutination, das eine leichte, direkte und verlängerte Anzeige ermöglicht,
**dadurch gekennzeichnet,**
daß nach der Inkubation eine Mischung von Serum und roten Blutkörperchen in ein Gelmilieu gegeben wird, das einen Antikörper oder keinen Antikörper enthält und diese Mischung besonderen Zentrifugationsbedingungen unterworfen wird, was in einem Schritt folgendes erlaubt:
a) die Trennung des Serums und der roten Blutkörperchen innerhalb des Testsystems,
b) den Kontakt der sensibilisierten oder nicht-sensibilisierten Blutkörperchen mit den enthaltenen Antikörpern im Gelmilieu,
c) das relative Zurückhalten der agglutinierten, roten Blutkörperchen durch das Gel im Verhältnis zu den nicht-agglutinierten, roten Blutkörperchen, welche sedimentieren.

2. Verfahren gemäß Anspruch 1,
**dadurch gekennzeichnet,**
daß das Gelmilieu durch Rekonstitution in einem physiologischen Puffer hergestellt wird.

3. Verfahren gemäß Anspruch 1,
**dadurch gekennzeichnet,**
daß das Gel auf Dextranbasis ist.

4. Verfahren gemäß Anspruch 2,
**dadurch gekennzeichnet,**
daß die Konservierung der Antikörper im Gel und die Trennung der agglutinierten und nicht-agglutinierten roten Blutkörperchen verbessert werden durch die Zugabe von Albumin, Dextranen, Polyvinylpyrrolidon oder Tierseren.

5. Verfahren gemäß Anspruch 2,
**dadurch gekennzeichnet,**
daß das Gel als Antikörper ein menschliches Antiglobulin enthält.

## Claims

1. Process for detection of an erythrocytic agglutination permitting an easy, direct and prolonged reading, characterized in that a mixture of red blood cell serum is incorporated, after incubation, into a gel medium containing antibodies or not, and in that this mixture is subjected to special conditions of centrifugation permitting at one stroke and at one time:
a) the separation of the serum and the red blood cells within the test system,
b) contact of the sensitized or non-sensitized red blood cells with the antibodies contained in the gel medium,
c) relative retention of the agglutinated red blood cells by the gel in relation to the non-agglutinated red blood cells which form sediment.

2. Process according to claim 1, characterized in that the gel medium is prepared by reconstitution in a physiological buffer.

3. Process according to claim 1, characterized in that the gel is dextran-base.

4. Process according to claim 2, characterized in that the conservation of antibodies in the gel and the separation of agglutinated and non-agglutinated red blood cells are improved through the addition of albumin, of dextran, of polyvinylpyrrolidone or animal serum.

5. Process according to claim 2, characterized in that the gel medium includes a human antiglobulin such as antibodies.
